(19) Europäisches Patentamt
European Patent Office
Office européen des brevets

(11) Numéro de publication: **0 170 549**
**B1**

(12) # FASCICULE DE BREVET EUROPÉEN

(45) Date de publication du fascicule du brevet: **25.01.89**

(21) Numéro de dépôt: **85401198.8**

(22) Date de dépôt: **18.06.85**

(51) Int. Cl.⁴: **C 07 D 471/04,** C 07 D 491/048, A 61 K 31/435 // C07D211/90 ,(C07D471/04, 221:00, 209:00),(C07D491/048, 307:00, 221:00)

(54) **Dérivés de tétrahydro-4,5,6,7 furo ou 1H-pyrrolo 2,3-c pyridine, leur préparation et leur application en therapeutique.**

(30) Priorité: **27.06.84 FR 8410108**

(43) Date de publication de la demande: **05.02.86 Bulletin 86/6**

(45) Mention de la délivrance du brevet: **25.01.89 Bulletin 89/4**

(84) Etats contractants désignés: **AT BE CH DE FR GB IT LI LU NL SE**

(56) Documents cité: **FR-A-2 215 948** **US-A-3 992 544**

(73) Titulaire: **SYNTHELABO, 58, rue de la Glacière, F-75621 Paris Cedex 13 (FR)**

(72) Inventeur: **Wick, Alexander, 10, Boulevard des Plants, F-78860 St Nom La Breteche (FR)**
Inventeur: **Frost, Jonathan, 7, Route de Montjean, F-91320 Wissous (FR)**
Inventeur: **Lardenois, Patrick, 18, rue Varengue, F-92340 Bourg La Reine (FR)**

(74) Mandataire: **Thouret- Lemaitre, Elisabeth, Service Brevets - SYNTHELABO 58, rue de la Glacière, F-75621 Paris Cédex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



LIBER, STOCKHOLM 1989

**Description**

La présente invention a pour objet des dérivés de tétrahydro-4,5,6,7 furo ou 1H-pyrrolo[2,3-c]pyridine.

Les composés de l'invention répondent à la formule générale I

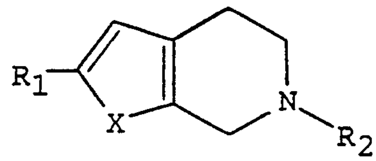

(I)

dans laquelle:

X représente un atome d'oxygène ou un groupe NH,

$R_1$ représente soit un groupe phényle portant éventuellement un ou deux atomes d'halogène ou un groupe méthyle, éthyle, trifluorométhyle, méthoxy, phényle ou, lorsque $R_2$ n'est pas un groupe benzyle, un groupe amino, soit un groupe naphtyle, soit un groupe benzodioxannyle, et

$R_2$ représente un atome d'hydrogène ou un groupe benzyle, ou bien encore, lorsque X représente un atome d'oxygène, $R_2$ peut représenter un groupe méthyle ou éthyle.

Ces composés (I) peuvent se présenter sous forme de bases libres ou de sels d'addition à des acides acceptables en pharmacologie, ces derniers faisant également partie de l'invention.

Les composés (I) que l'on préfère sont ceux dans la formule desquels $R_2$ représente l'hydrogène.

Une autre catégorie de composés préférés est celle des composés dans la formule desquels X représente un atome d'oxygène et $R_2$ représente un groupe méthyle ou éthyle.

Des dérivés de pyrrolopyridines et des dérivés de furopyridines sont décrits dans le brevet des Etats Unis d'Amérique 3 992 544 et dans la demande de brevet français 2 215 948, respectivement. Ces composés connus, de configuration [3,2-c] au lieu de [2,3-c], sont par ailleurs bien différents de ceux de l'invention, notamment en ce qui concerne les substituants en position 2. En outre leurs indications thérapeutiques sont autres que celles des composés de l'invention.

Les composés de l'invention peuvent être préparés conformément au schéma donné ci-après.

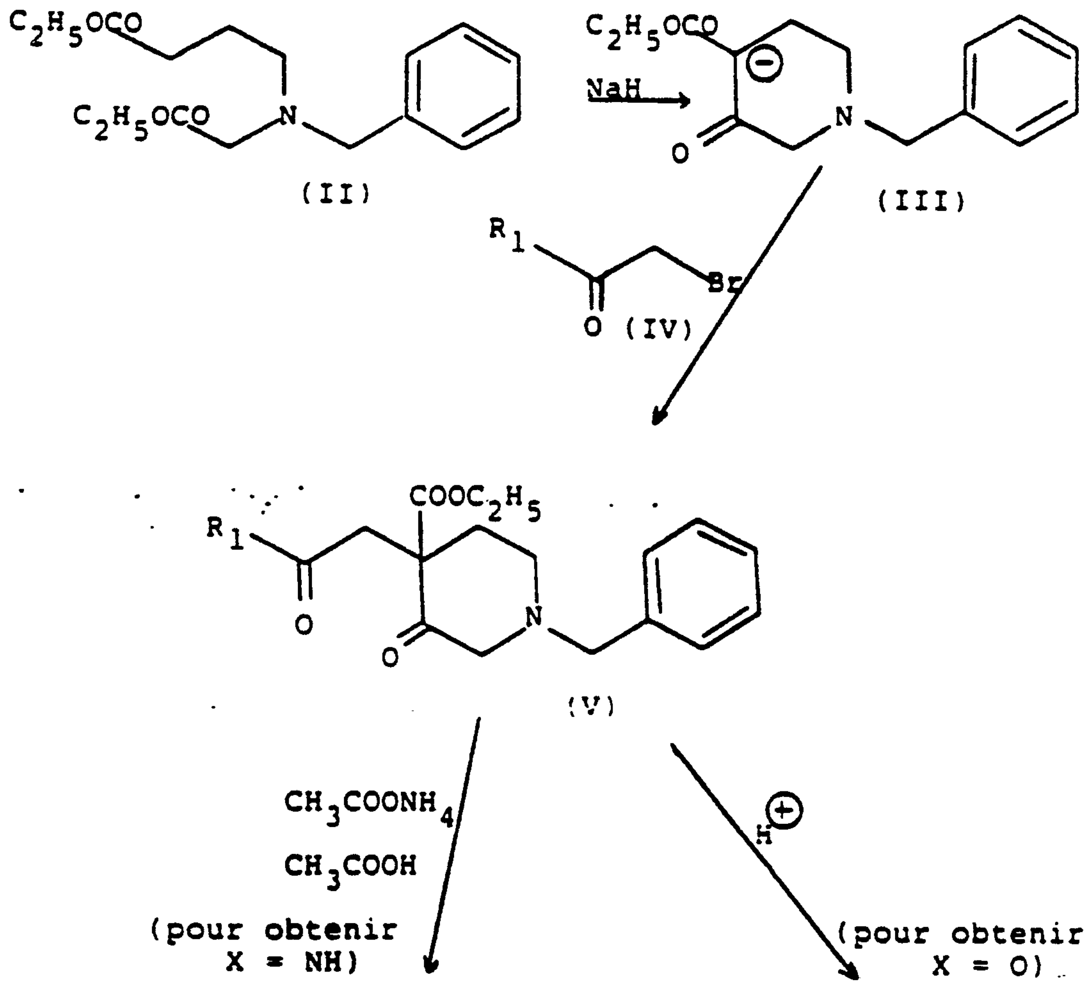

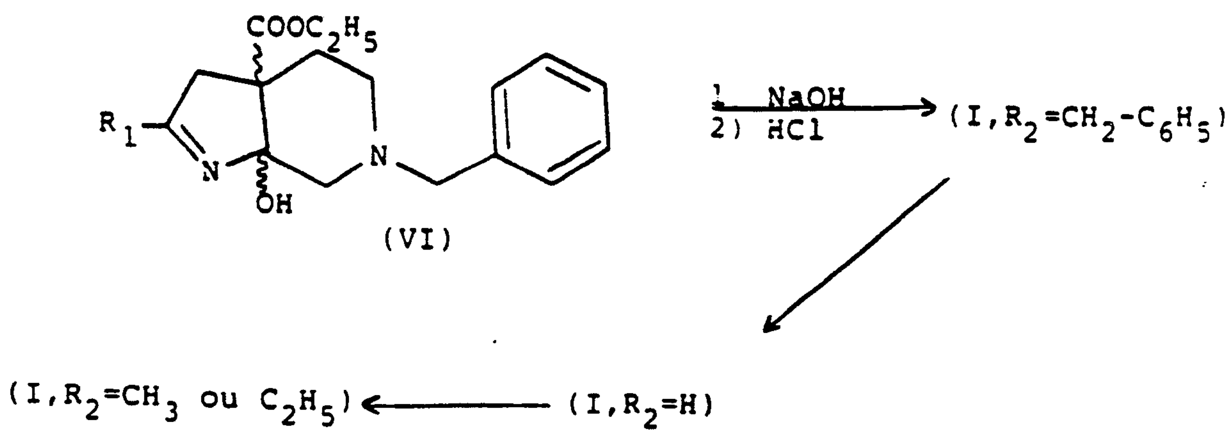

(VI)

La première étape du procédé consiste à cycliser un diester de formule II, de préférence l'ester diéthylique, en présence d'un hydrure alcalin, à la température de reflux d'un solvant tel que le toluène.

Le composé de formule III ainsi obtenu est ensuite mis à réagir avec une acétophénone de formule IV portant un groupe labile tel qu'un atome de brome, et convenablement substituée par $R_1$ sur le noyau benzénique. Cette réaction s'effectue de préférence à température ambiante.

Selon le composé (I) à préparer, c'est-à-dire selon que X représente 0 ou NH, le composé de formule V obtenu est alors soumis à deux types de réactions.

Si l'on souhaite préparer un dérivé de pyrrolopyridine (X=NH), on effectue d'abord une cyclisation en présence d'acétate d'ammonium et d'acide acétique, puis on décarboxyle et on déshydrate le composé de formule VI résultant en le traitant d'abord par de la soude puis par de l'acide chlorhydrique.

Si l'on souhaite préparer un dérivé de furopyridine (X=0), on fait subir au composé (V) une cyclisation en milieu acide fort.

Les composés de formule I ainsi obtenus portent nécessairement un substituant $R_2$ qui est un groupe benzyle. C'est pourquoi, si on le désire, on peut effectuer une débenzylation pour obtenir un composé (I) ou $R_2$ est l'hydrogène, par exemple en effectuant une hydrogénation catalytique ou encore en faisant d'abord réagir le composé (I) benzylé avec du chloroformiate de trichloro-2,2,2 éthyle et en fragmentant le carbamate obtenu par le zinc en présence d'acide acétique.

Pour préparer les composés dans la formule I desquels $R_1$ représente un groupe aminophényle, on utilise une acétophénone de formule IV, dans laquelle $R_1$ est un groupe nitrophényle. Le groupe nitro ainsi introduit est réduit en groupe amino au cours de la débenzylation finale par hydrogénation catalytique (groupe benzyle $R_2$ remplacé par un atome d'hydrogène).

Le composé de départ de formule II peut être préparé par exemple par action du bromo-4 butanoate d'éthyle sur le phénylméthylaminoacétate d'éthyle en présence d'une base telle que la diméthyl-2,6 pyridine.

Dans le cas des composés de formule I dans laquelle X représente un atome d'oxygène et $R_2$ représente un atome d'hydrogène, on peut, au lieu du dérivé de départ N-benzylé de formule II, utiliser le dérivé N-benzoylé correspondant. Le groupe benzoyle s'élimine ensuite au cours de la réaction de cylisation en milieu acide du composé de formule V correspondant (c'est-à-dire benzoylé au lieu de benzylé), et une débenzylation finale n'a plus lieu d'être.

Enfin, les composés (I) dans la formule desquels $R_2$ représente un groupe méthyle ou éthyle s'obtiennent par alkylation à partir des composés où $R_2$ est l'hydrogène, par exemple par action d'un halogénure de méthyle ou d'éthyle, ou encore au moyen d'acide formique et de formaldéhyde (dans le cas du groupe méthyle), ou par acétylation puis réduction (dans le cas du groupe éthyle).

Les exemples suivants illustrent l'invention.

Les structures des composés préparés sont confirmées par les micro-analyses et les spectres IR et RMN.

**Exemple 1.**

**Phényl-2 phénylméthyl-6 tétrahydro-4,5,6,7 1H-pyrrolo [2,5-c]pyridine et son chlorhydrate.**

a) A une solution de 20 g (0,065 mole) de [(éthoxy-2 oxo-2 éthyl)(phénylméthyl)]amino-4 butanoate d'éthyle dans 440 ml de toluène on ajoute 3,25 g (0,065 mole) d'une suspension d'hydrure de sodium à 50 % dans de l'huile minérale, on chauffe au reflux et on ajoute quelques gouttes d'éthanol anhydre.

La réaction est exothermique, et on obtient une solution qu'on maintient encore 4 heures au reflux, puis on la refroidit sur bain de glace.

b) On ajoute à la solution précédemment préparée 14 g (0,070 mole) de bromo-1 acétophénone et on agite le mélange pendant une nuit à température ambiante.

On ajoute 200 ml d'eau, on agite, et on sépare la phase organique que l'on sèche et évapore.

Il reste une huile qu'on utilise telle quelle dans l'étape suivante.

c) A 24,6 g (0,065 mole) de l'huile précédemment obtenue on ajoute une solution de 15 g (0,195 mole) d'acétate d'ammonium dans 120 ml d'acide acétique et on agite le mélange pendant 5 heures à température ambiante.

On le verse sur un mélange refroidi de 300 ml d'eau et de glace, 160 ml d'ammoniaque concentrée et 50 ml d'acétate d'éthyle.

On agite le tout pendant 10 minutes à température ambiante, on filtre le précipité formé, on le lave à l'éthanol et on le sèche.

d) On introduit 10,4 g du solide précédemment obtenu dans un mélange de 200 ml d'eau, 200 ml d'éthanol et 200 ml de lessive de soude, et on agite à température ambiante pendant 3 heures.

On sépare un léger louche par filtration et, sur bain de glace, on ajoute au filtrat, goutte à goutte, 200 ml d'acide chlorhydrique concentré.

On agite pendant 2 heures à température ambiante, puis on filtre le précipité, on le lave à l'eau et à l'éthanol, on le sèche, on le recristallise dans l'éthanol à 95 % et on le sèche. Point de fusion: 236 - 238° C.

**Exemple 2**

**(Méthoxy-4 phényl)-2 phénylméthyl-6 tétrahydro-4,5,6,7 1H-pyrrolo[2,3-c]pyridine et son chlorhydrate.**

a) On prépare une solution de 0,1 mole de l'anion du oxo-3 phénylméthyl-1 pipéridinecarboxylate-4 d'éthyle selon l'exemple 1a) ci-dessus, dans 700 ml de toluène.

b) On ajoute à la solution ainsi préparée 22,9 g (0,1 mole) de bromo-1 méthoxy-4'acétophénone et on agite une nuit à température ambiante.

On ajoute 100 ml d'eau, on agite 15 minutes, on sépare la phase organique, on la sèche et on l'évapore. Il reste une huile qu'on utilise telle qu'elle dans l'étape suivante.

c) A 26,6 g (0,065 mole) de l'huile précédemment obtenue on ajoute une solution de 15 g (0,195 mole) d'acétate d'ammonium dans 120 ml d'acide acétique. On termine la préparation comme indiqué à l'exemple 1c) ci-dessus et on recueille un solide que l'on utilise tel quel.

d) On introduit 8,4 g (0,0205 mole) de ce solide dans un mélange de 170 ml d'eau, 170 ml d'éthanol et 170 ml de lessive de soude, et on agite le mélange pendant 3 h 30 à température ambiante.

On le refroidit sur un bain de glace et on ajoute goutte à goutte 170 ml d'acide chlorhydrique concentré et on laisse agir une nuit à température ambiante.

On filtre le précipité, on le lave à l'eau puis à l'acétone, on le sèche et on le recristallise dans un mélange éthanol/eau. Après séchage il reste des cristaux de couleur légèrement bleu-vert. Point de fusion: 237 - 238° C.

**Exemple 3**

**(Bromo-4 phényl)-2 phénylméthyl-6 tétrahydro-4,5,6,7 1H-pyrrolo[2,3-c]pyridine et son chlorhydrate.**

a) On prépare une solution de 0,2 mole de l'anion du oxo-3 phénylméthyl-1 pipéridinecarboxylate-4-d'éthyle selon l'exemple 1a) ci-dessus.

b) A la solution ainsi préparée on ajoute 55,6 g (0,2 mole) de dibromo-1,4' acétophénone et on laisse réagir pendant une nuit.

Ensuite on ajoute 200 ml d'eau, on agite, on sépare la phase organique par décantation, on la sèche et on l'évapore. Il reste une huile.

c) On en introduit 45,8 g (0,1 mole) dans une solution de 23 g (0,3 mole) d'acétate d'ammonium et 180 ml d'acide acétique, et on agite à température ambiante pendant 3 heures. Puis, tout en refroidissant, on verse le mélange sur une solution de 600 ml d'eau et de glace, 280 ml d'ammoniaque concentrée et 50 ml d'acétate

d'éthyle.

On agite quelques minutes, on filtre le précipité, on le lave à l'éther de pétrole et on le sèche. Point de fusion 160° C environ.

d) On en introduit 18,7 g (0,041 mole) dans un mélange de 180 ml d'éthanol, 180 ml d'eau et 180 ml de lessive de soude, on agite le mélange pendant 6 heures à température ambiante, on filtre un léger louche, on refroidit le mélange et on ajoute goutte à goutte 180 ml d'acide chlorhydrique concentré.

Après 45 minutes d'agitation on filtre le précipité, on le lave à l'eau puis à l'éther, on le sèche, on le recristallise dans un mélange 50/50 d'éthanol/eau, et on le sèche. Point de fusion: 264 - 266°C, avec décomposition.

**Exemple 4**

**(Fluoro-4 phényl)-2 phénylméthyl-6 tétrahydro-4,5,6,7 1H-pyrrolo[2,3-c]pyridine et son chlorhydrate.**

a) On prépare une solution de l'anion du oxo-3 phénylméthyl-1 pipéridinecarboxylate-4 d'éthyle à partir de 30,7 g (0,1 mole) de [(éthoxy-2 oxo-2 éthyl) (phénylméthyl)]amino-4 butanoate d'éthyle, selon la méthode exposée à l'exemple 1a) ci-dessus.

b) A la solution refroidie on ajoute 21,7 g (0,1 mole) de bromo-1 fluoro-4' acétophénone, et on laisse réagir une nuit à température ambiante.

On sépare la phase organique, on la lave, on la sèche et on l'évapore. Il reste une huile que l'on utilise telle quelle.

c) On introduit 19,9 g (0,05 mole) de cette huile dans une solution de 11,5 g d'acétate d'ammonium dans 95 ml d'acide acétique et on agite le mélange pendant 3 h 30 à température ambiante.

On le verse ensuite sur une solution de 300 ml de glace, 140 ml d'ammoniaque concentrée et 25 ml d'acétate d'éthyle. On agite 15 minutes en refroidissant, on filtre le précipité, on le lave à l'eau puis à l'éther et on le sèche. Point de fusion: 176° C.

d) A 10,5 g (0,0264 mole) de ce solide on ajoute 100 ml d'éthanol, 100 ml d'eau et 100 ml de lessive de soude, et on laisse réagir une nuit à température ambiante.

Puis on ajoute à la solution obtenue, goutte à goutte, 100 ml d'acide chlorhydrique concentré, et on agite encore 2 heures.

On filtre le précipité, on le lave à l'eau et à l'éther, on le sèche, on le recristallise dans 350 ml d'un mélange éthanol/eau à 1 % d'acide chlorhydrique concentré et on le sèche. Point de fusion: 246 - 248° C.

**Exemple 5**

**(Méthoxy-4 phényl)-2 tétrahydro-4,5,6,7 1H-pyrrolo[2,3-c] pyridine et son chlorhydrate.**

On prépare un mélange de 7,1 g (0,02 mole) du composé obtenu selon l'exemple 2, 70 ml d'acide acétique, 70 ml d'eau et 0,7 g de charbon palladié à 10 %, et on le soumet pendant 7 heures à une hydrogénation sous une pression de 0,35 MPa à 50° C.

Après une nuit de repos on replace sous hydrogénation dans les mêmes conditions pendant 5 heures.

On sépare le catalyseur, on évapore le filtrat, on reprend le résidu cristallisé avec 10 ml d'éthanol à 10 % d'acide chlorhydrique concentré, on l'agite 15 minutes sur bain de glace, on filtre le solide, on le lave à l'éther, on le recristallise dans 100 ml d'éthanol à 1 % d'acide chlorhydrique concentré et on le sèche. Point de fusion: 235° C avec décomposition.

**Exemple 6**

**Phényl-2 tétrahydro-4,5,6,7 1H-pyrrolo[2,3-c]pyridine et son chlorhydrate.**

A 1 g (0,003 mole) du chlorhydrate obtenu selon l'exemple 1 on ajoute 10 ml d'acide acétique, 10 ml d'eau et 100 mg de charbon palladié à 5 % et on soumet le mélange à une hydrogénation sous 0,35 MPa pendant 7 heures à 50° C.

On filtre le catalyseur, on évapore le filtrat, on reprend le résidu solide avec 10 ml d'éthanol à 1 % d'acide chlorhydrique concentré, on filtre le précipité, on le lave avec de l'éther on le sèche, on en libère la base dans de l'acétate d'éthyle au moyen d'ammoniaque et on la purifie par chromatographie sur silice en éluant avec un mélange 8/2 de chloroforme/méthanol. Point de fusion: 179 - 181° C.

On en prépare le chlorhydrate dans de l'éthanol. Point de fusion: 249 - 251° C.

**Exemple 7**

**(Méthoxy-4 phényl)-2 phénylméthyl-6 tétrahydro-4,5,6,7 furo[2,3-c]pyridine et son chlorydrate.**

a) On prépare l'anion du oxo-3 phénylméthyl-1 pipéridinecarboxylate-4 d'éthyle selon l'exemple 1a) ci-dessus.

b) On prépare le [(méthoxy-4 phényl)-2 oxo-2 éthyl]-4 oxo-3 phénylméthyl-1 pipéridinecarboxylate-4 d'éthyle selon l'exemple 2b) ci-dessus.

c) On dissout 18 g (0,044 mole) de l'huile ainsi obtenue dans 18 ml d'éthanol, on ajoute 270 ml d'acide chlorhydrique concentré, et on chauffe au reflux pendant 1 h 30, puis on refroidit le mélange sur de la glace.

On filtre le précipité formé, on le lave à l'eau puis à l'éther puis au chloroforme, on le recristallise dans un mélange 75/25 d'éthanol/eau et on le sèche. Point de fusion: 252 - 253°C.

**Exemple 8**

**(Méthyl-4 phényl)-2 phénylméthyl-6 tétrahydro-4,5,6,7 furo[2,3-c]pyridine et son chlorhydrate.**

a) On prépare l'anion du oxo-3 phénylméthyl-1 pipéridinecarboxylate-4 d'éthyle selon l'exemple 1a) ci-dessus.

b) On refroidit la solution obtenue, on ajoute 21,3 g (0,1 mole) de bromo-1 méthyl-4' acétophénone en solution dans 50 ml de toluène et on laisse agir une nuit à température ambiante.

On ajoute 100 ml d'eau, on agite, on sépare la phase organique et on l'évapore. Il reste un huile qu'on utilise telle quelle.

c) On dissout l'huile ainsi obtenue dans 40 ml d'éthanol, on ajoute 500 ml d'acide chlorhydrique concentré, on chauffe au reflux pendant 1 h 30 et on refroidit le mélange sur de la glace.

On filtre le précipité formé, on le lave au chloroforme puis à l'éther, on le sèche, on le recriatallise dans de l'éthanol. Il reste un produit blanc. Point de fusion: 251 - 253°C.

**Exemple 9**

**(Bromo-4 phényl)-2 phénylméthyl-6 tétrahydro-4,5,6,7 furo[2,3-c]pyridine et son chlorhydrate.**

a) On prépare une solution de l'anion du oxo-3 phénylméthyl-1 pipéridinecarboxylate-4 d'éthyle selon la méthode exposée à l'exemple 1a) ci-dessus.

b) On prépare le [(bromo-4 phényl)-2 oxo-2 éthyl]-4 oxo-3 phénylméthyl-1 pipéridineoarboxylate-4 d'éthyle selon la méthode exposée à l'exemple 3b) ci-dessus.

c) On dissout 45,8 g (0,1 mole) de l'huile ainsi obtenue dans 40 ml d'éthanol, on ajoute 500 ml d'acide chlorhydrique concentré et on chauffe au reflux pendant 2 h 30.

On refroidit le mélange, on filtre le précipité marron, on le lave au chloroforme puis à l'éther, on le recristallise dans un mélange 1/1 d'éthanol/eau et on le sèche. Il reste un produit blanc. Point de fusion: 263 - 265°C avec décomposition.

**Exemple 10**

**(Méthoxy-4 phényl)-2-tétrahydro-4,5,6,7 furo[2,3-c]pyridine et son chlorhydrate.**

On prépare une suspension de 11 g (0,309 mole) du composé préparé selon l'exemple 7 dans 100 ml d'acide acétique et 100 ml d'eau, on ajoute 250 mg de charbon palladié à 5 %, et on soumet le mélange à une hydrogénation sous environ 0,35 MPa pendant 7 heures à 50°C.

On filtre le catalyseur, on évapore le filtrat, on reprend le résidu cristallisé avec 25 ml d'éthanol à 1 % d'acide chlorhydrique concentré, on en libère la base pour la purifier par chromatographie sur silice en éluant avec un mélange 8/2 de chloroforme/méthanol. Le base pure fond à 112°C.

On en prépare le chlorydrate dans 100 ml d'éthanol à 1 % d'acide chlorhydrique concentré, on chauffe à reflux pour recristalliser le sel, on le filtre et on le sèche. Point de fusion: 226 - 228°C.

**Exemple 11**

**(Bromo-4 phényl)-2 tétrahydro-4,5,6,7 furo[2,3-c]pyridine et son chlorhydrate.**

On introduit 5,8 g (0,0157 mole) du composé préparé selon l'exemple 9 dans 120 ml de toluène, on ajoute 6,35 ml (0,0471 mole) de chloroformiate de trichloro-2,2,2 éthyle et on chauffe au reflux pendant 12 heures. On laisse reposer une nuit, on ajoute encore 2 ml de chloroformiate de trichloro-2,2,2 éthyle et on reporte au reflux pendant 4 heures.

On chasse le solvant et on reprend le (bromo-4 phényl)-2 tétrahydro-4,5,6,7 furo[2,3-c]pyridinecarboxylate-6 de trichloro-2,2,2 éthyle résiduel par 75 ml d'acide acétique, on ajoute 5 g de poudre de zinc, on agite pendant 7 heures et on laisse reposer.

On filtre, on évapore le filtrat, on le reprend avec 100 ml d'eau et on extrait à l'acétate d'éthyle après avoir ajouté de l'ammoniaque. On évapore les phases organiques on les purifie sur silice avec un mélange 8/2 de chloroforme/méthanol, on dissout la base purifiée dans 5 ml d'éthanol et on ajoute quelques gouttes d'acide chlorhydrique concentré.

On filtre le précipité et on le recristallise dans de l'éthanol à 1 % d'acide chlorhydrique concentré. Point de fusion: 274 - 275° C.

**Exemple 12**

**(Méthyl-4 phényl)-2 tétrahydro-4,5,6,7 furo[2,3-c]pyridine et son chlorhydrate.**

On introduit 8,5 g (0,025 mole) du composé préparé selon l'exemple 8 dans une solution de 100 ml d'acide acétique et 100 ml d'eau, on ajoute 250 mg de charbon palladié à 5 % et on soumet l'ensemble à une hydrogénation sous environ 0,35 MPa pendant 7 heures à 50° C.

On sépare le catalyseur, on évapore le filtrat, on libère la base du résidu avec de l'ammoniaque 1 N dans de l'acétate d'éthyle, on décante et on évapore la phase organique, on purifie la base en éluant sur silice avec un mélange de chloroforme/méthanol, on la dissout dans 40 ml d'éthanol et on ajoute 40 ml d'éthanol à 1 % d'acide chlorhydrique concentré. On recristallise le précipité et on le sèche. Point de fusion: 250 - 252° C.

**Exemple 13**

**(Amino-4 phényl)-2 tétrahydro-4,5,6,7 furo[2,3-c]pyridine et son chlorhydrate.**

a) On prépare l'anion du oxo-3 phénylméthyl-1 pipéridinecarboxylate-4 d'éthyle selon l'exémple 1a) ci-dessus, à partir de 30,7 g (0,1 mole) de [(éthoxy-2 oxo-2 éthyl)(phénylméthyl)]amino-4 butanoate d'éthyle dans 700 ml de toluène et de 5 g (0,1 mole) d'hydrure de sodium à 50 %.

b) On refroidit la solution ainsi obtenue et on ajoute 24,4 g (0,1 mole) de bromo-1-nitro-4' acétophénone et on agite le mélange une nuit à température ambiante. Puis on le lave avec 200 ml d'eau, on filtre un léger précipité, on sépare la phase organique et on l'évapore. Il reste une huile.

c) On ajoute à cette huile 40 ml d'éthanol et 500 ml d'acide chlorhydrique concentré et on chauffe le mélange au reflux et, après 1 h 30, on refroidit et on isole une première fraction de précipité. On réchauffe au reflux pendant 1 h et on sépare une deuxième fraction de précipité et, après un troisième réchauffage, on recueille une troisième fraction.

On réunit les trois lots et on les lave à l'acétone puis à l'éther, et on les sèche. Point de fusion: 254° C.

d) On introduit 5,6 g du composé précédent dans un mélange de 55 ml d'acide acétique et 55 ml d'eau, on ajoute 560 mg de charbon palladié à 5 % et on effectue une hydrogénation sous 0,35 MPa à température ambiante pendant 15 mn, puis à 50° C pendant 1 h. On sépare le catalyseur par filtration, on évapore le filtrat et on reprend le résidu avec 100 ml d'éther. On ajoute de l'ammoniaque pour libérer la base, on recueille la phase organique, on la sèche, on l'évapore et on purifie l'huile résiduelle par chromatograhie sur silice, en éluent avec un mélange 8/2 de dichlorométhane/méthanol. Le base purifiée fond à 160° C. On la dissout dans un mélange de 30 ml d'éthanol et 20 ml d'éther, on filtre un léger louche, et on ajoute 0,5 ml d'acide chlorhydrique concentré, en agitant pendant 15 mn. On filtre le précipité formé, on le lave à l'éther et on le recristallise dans un mélange de 40 ml d'éthanol et 15 ml d'eau, avec 1 % d'acide chlorhydrique concentré, on recueille les cristaux formés et on les sèche. Point de fusion: 258 - 260° C.

**Exemple 14**

**(Chloro-4 phényl)-2 tétrahydro-4,5,6,7 furo[2,3-c]pyridine et son chlorhydrate.**

a) On dissout 85,1 g, soit 76 ml (1 mole) de pyrrolidone-2 dans 700 ml de toluène et on ajoute 50 g (1 mole) d'hydrure de sodium à 50 % dans de l'huile minérale par fraction de 10 g en 1 h au bain de glace. On agite

encore 1 h à température ambiante puis, de nouveau au bain de glace, on ajoute lentement, en 30 mn, 106 ml (1 mole) de chloroacétate d'éthyle en solution dans 200 ml de toluène. On maintient l'agitation pendant 1 h, puis on ajoute 300 ml d'eau. On agite le mélange, on sépare la phase organique, on la lave avec 200 ml d'eau, on la sèche et on l'évapore. Il reste une huile.

b) On chauffe au reflux pendant 6 h un mélange de 110 g de l'huile précédente, 250 ml d'acide chlorhydrique concentré, et 250 ml d'eau. On laisse reposer une nuit, on chauffe encore pendant 8 h au reflux, et on laisse reposer le mélange pendant deux jours.

On lave la solution à l'éther de pétrole, on l'évapore et on la sèche. On reprend le résidu sec avec 400 ml d'éthanol, on sature le mélange avec du chlorure d'hydrogène gazeux et on le chauffe au reflux pendant 5 h. Après une nuit de repos on évapore l'alcool. Il reste le chlorure de (éthoxy-2 oxo-2 éthyl)amino-4 butanoate d'éthyle sous forme d'huile sirupeuse.

c) On agite vigoureusement une solution de 150 g de carbonate de potassium dans 200 ml d'eau, placée dans un bain de glace, et on y verse lentement l'huile précédente. Puis on ajoute 100 ml d'eau et on extrait la base libérée deux fois avec 200 ml d'éther. On réunit les phases organiques, on les lave, on les sèche et on chasse l'éther.

On dissout l'huile résiduelle dans 200 ml de pyridine et on ajoute goutte à goutte 40 ml de chlorure de benzoyle, tout en maintenant la température inférieure à 30° C.

Après l'addition on chauffe le mélange pendant 1 h à 80° C, puis on évapore le mélange, on reprend le résidu avec 200 ml de solution aqueuse 1N d'acide chlorhydrique et on l'extrait à l'acétate d'éthyle. Après lavage, séchage et évaporation de la phase organique, il reste le [(éthoxy-2 oxo-2 éthyl) (phénylcarbonyl)]amino-4 butanoate d'éthyle sous forme d'huile.

d) On en dissout 16 g dans 160 ml de toluène anhydre, on ajoute 2,5 g d'hydrure de sodium à 50 % dans l'huile et on chauffe au reflux. Après 10 mn on ajoute goutte à goutte 10 ml d'éthanol anhydre, et on chauffe la solution au reflux pendant 7 h, puis on la refroidit dans un bain de glace.

On ajoute alors 11,7 g de bromo-1 chloro-4' acétophénone et on agite une nuit à température ambiante.

On lave le mélange avec 30 ml d'eau, on sépare la phase organique, on l'évapore, on reprend le résidu par 20 ml d'éthanol et 250 ml d'acide chlorhydrique concentré, et on chauffe pendant 2 h 30 au reflux. Puis on refroidit dans un bain de glace, tout en agitant, ce qui provoque la formation d'un précipité marron. On filtre ce dernier, on le reprend avec 50 ml d'acétone et on l'agite dans de la glace. On sépare le solide, on chasse l'acétone, on reprend le résidu avec 50 ml d'acétate d'éthyle, on le sépare et on l'ajoute au premier solide recueilli. On le sèche puis on le recristallise dans de l'éthanol à 98 % contenant 1 % d'acide chlorhydrique concentré, et on le sèche. Point de fusion: 267 - 269° C.

**Exemple 15**

**(Méthyl-4 phényl)-2 méthyl-6 tétrahydro-4,5,6,7 furo[2,3-c] pyridine et son chlorhydrate.**

On introduit 4 g du composé obtenu selon l'exemple 12 dans un mélange d'acétate d'éthyle et d'ammoniaque, et on libère la base en agitant le mélange, isolant la phase organique et évaporant le solvant.

On introduit 1,75 g du résidu d'évaporation dans un mélange de 3 ml d'acide formique et 3 ml de formaldéhyde à 40 %, et on chauffe à 100° C au bain d'huile pendant 30 mn. On laisse refroidir, et on verse le mélange sur 100 ml de glace, et on l'extrait à l'acétate d'éthyle. Après lavage, séchage et évaporation du solvant il reste une huile qu'on purifie par chromatographie sur colonne de silice en éluant avec de l'acétone. On dissout la base pure dans 40 ml d'éther, on sépare un léger louche par filtration et on ajoute au filtat de l'éthanol saturé de gaz chlorhydrique. On agite 10 mn à température ambiante, on filtre le précipité et on le recristallise dans 40 ml d'éthanol auxquels on ajoute quelques gouttes d'eau jusqu'à dissolution complète. Après recristallisation on lave le produit à l'éther et on le sèche. Point de fusion: 270 - 272° C.

**Exemple 16**

**(Bromo-4 phényl)-2 éthyl-6 tétrahydro-4,5,6,7 furo[2,3-c] pyridine et son chlorhydrate.**

On libère la base du composé de l'exemple 11 et on en introduit 2,2 g dans 25 ml d'anhydride acétique et on agite une nuit à température ambiante. On verse la suspension obtenue sur 150 ml de glace, on extrait à l'acétate d'éthyle, on lave la phase organique à l'ammoniaque puis à l'eau, on l'évapore et on sèche le résidu par entraînement azéotropique au toluène.

On prépare une suspension de 0,25 g de chlorure d'aluminium et 0,5 g d'hydrure d'aluminium et de lithium dans 25 ml de tétrahydrofuranne anhydre, on la refroidit dans un bain de glace, et on ajoute goutte à goutte une solution de 2,4 g du résidu sec précédent dans 25 ml de tétrahydrofuranne sec. Après le fin de l'addition on

agite encore 15 mn puis on ajoute lentement 5 ml d'eau puis 100 ml d'acétate d'éthyle.

On ajoute ensuite de l'eau par petites fractions jusqu'à empâtage des hydroxydes d'aluminium et de lithium, on sépare la phase organique par filtration et on l'évapore. Il reste un résidu solide qu'on sèche par entraînement azéotropique au toluène, puis qu'on purifie par chromatographie sur colonne de silice en éluant avec un mélange 8/2 de dichlorométhane/méthanol. On dissout la base purifiée dans 50 ml d'éther et on ajoute 3 ml d'éthanol saturé de chlorure d'hydrogène. On agite 10 mn, on filtre le précipité, on le recristallise dans l'éthanol absolu, on le lave à l'éther et on le sèche. Point de fusion: 262 - 264°C.

Le tableeu ci-après illustre les structures et les propriétés physiques de divers composés selon l'invention.

**Tableau**

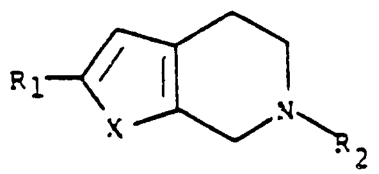

(I)

| Composé | Exemple | X | R1 | R2 | Base/Sel | F(°C) |
|---|---|---|---|---|---|---|
| 1 | 1 | NH | $C_6H_5$- | -$CH_2$-$C_6H_5$ | HCl | 236 - 238 |
| 2 | 2 | NH | 4-$CH_3O$-$C_6H_4$- | -$CH_2$-$C_6H_5$ | HCl | 237 - 238 |
| 3 | | NH | 4-$CH_3$-$C_6H_4$- | -$CH_2$-$C_6H_5$ | HCl | 246 - 248 |
| 4 | 3 | NH | 4-Br-$C_6H_4$- | -$CH_2$-$C_6H_5$ | HCl | 264 - 266 |
| 5 | | NH | 3-$CH_3O$-$C_6H_4$- | -$CH_2$-$C_6H_5$ | HCl | 216 - 218 |
| 6 | | NH | 4-Cl-$C_6H_4$- | -$CH_2$-$C_6H_5$ | HCl | 257 - 259 |
| 7 | 4 | NH | 4-F-$C_6H_4$- | -$CH_2$-$C_6H_5$ | HCl | 246 - 248 |
| 8 | | NH | naphtyl-2 | -$CH_2$-$C_6H_5$ | HCl | 236 - 238 |
| 9 | 5 | NH | 4-$CH_3O$-$C_6H_4$ | H | HCl | 235 |
| 10 | 6 | NH | $C_6H_5$- | H | Base | 179 - 181 |
| | | | | | HCl | 249 - 251 |
| 11 | | O | $C_6H_5$- | -$CH_2$-$C_6H_5$ | HCl | 244 - 245 |
| 12 | 7 | O | 4-$CH_3O$-$C_6H_4$- | -$CH_2$-$C_6H_5$ | HCl | 252 - 253 |
| 13 | 8 | O | 4-$CH_3$-$C_6H_4$- | -$CH_2$-$C_6H_5$ | HCl | 251 - 253 |
| 14 | | O | 3-$CH_3O$-$C_6H_4$- | -$CH_2$-$C_6H_5$ | HCl | 219 - 221 |
| 15 | 9 | O | 4-Br-$C_6H_4$- | -$CH_2$-$C_6H_5$ | Hcl | 263 - 265 |
| 16 | | O | 4-Cl-$C_6H_4$- | -$CH_2$-$C_6H_5$ | Hcl | 265 - 267 |
| 17 | | O | 4-F-$C_6$-$H_4$- | -$CH_2$-$C_6H_5$ | HCl | 248 - 250 |
| 18 | | O | $C_6H_5$ | H | HCl | 255 - 257 |
| 19 | | O | 4-$C_6H_5$-$C_6H_4$- | -$CH_2$-$C_6H_5$ | HCl | 270 - 272 |
| 20 | 10 | O | 4-$CH_3O$-$C_6H_4$- | H | HCl | 226 - 228 |
| 21 | 11 | O | 4-Br-$C_6H_4$- | H | HCl | 274 - 275 |
| 22 | 12 | O | 4-$CH_3$-$C_6H_4$- | H | HCl | 250 - 252 |
| 23 | | O | nephtyl-2 | -$CH_2$-$C_6H_5$ | HCl | 249 - 251 |
| 24 | | O | 3,4-$Cl_2$-$C_6H_3$- | H | HCl | 261 - 263 |
| 25 | | O | naphtyl-2 | H | HCl | 265 - 267 |
| 26 | | O | 4-F-$C_6H_4$- | H | HCl | 254 - 255 |
| 27 | | O | 3-$CH_3$-$C_6H_4$- | H | HCl | 257 - 259 |
| 28 | | O | 2-$CH_3$-$C_6H_4$- | H | HCl | 245 - 247 |
| 29 | | O | 3-$CF_3$-$C_6H_4$- | H | HCl | 273 - 275 |
| 30 | | O | 2-F-$C_6H_4$- | H | HCl | 284 - 286 |
| 31 | | O | 3-F-$C_6H_4$- | H | HCl | 258 - 260 |
| 32 | 14 | O | 4-Cl-$C_6H_4$- | H | HCl | 267 - 269 |
| 33 | | O | 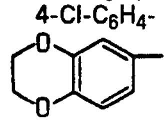 | H | HCl | 260 - 262 |
| 34 | | O | 4-$C_2H_5$-$C_6H_4$- | H | HCl | 235 - 237 |
| 35 | 13 | O | 4-$NH_2$-$C_6H_4$- | H | HCl | 258 - 260 |
| 36 | | O | 3-$C_2H_5$-$C_6H_4$- | H | HCl | 225 - 226 |
| 37 | | O | 3-$NH_2$-$C_6H_4$- | H | 2HCl | > 300 |
| 38 | 15 | O | 4-$CH_3$-$C_6H_4$- | -$CH_3$ | HCl | 270 - 272 |
| 39 | | O | 4-$CH_3$-$C_6H_4$- | -$CH_2CH_3$ | HCl | 250 - 252 |
| 40 | | O | $C_6H_5$- | -$CH_3$ | HCl | 263 - 265 |

| | | | | | | |
|---|---|---|---|---|---|---|
| 41 | | O | $C_6H_5$- | $-CH_2CH_3$ | HCl | 237 - 239 |
| 42 | | O | 4-F-$C_6H_4$- | $-CH_3$ | HCl | 250 - 252 |
| 43 | | O | 4-F-$C_6H_4$- | $-CH_2CH_3$ | HCl | 263 - 265 |
| 44 | | O | 4-Br-$C_6H_4$- | $-CH_3$ | HCl | 291 - 293 |
| 45 | 16 | O | 4-Br-$C_6H_4$- | $-CH_2CH_3$ | HCl | 262 - 264 |

Les composés de l'invention ont été soumis à des essais pharmacologiques.

La toxicité (dose létale 50, DL 50) des composés a été déterminée chez des souris de souche CD1 par méthode graphique. Les DL 50 vont de 100 à de 1000 mg/kg par voie intrepéritonéale.

Les composés de l'invention ont été soumis au test de l'ischémie cérébrale complète. L'ischémie est due à un arrêt cardiaque induit par une injection intraveineuse rapide de $MgCl_2$. Dans ce test on mesure le "temps de survie", c'est-à-dire l'intervalle entre le moment de l'injection de $MgCl_2$ et le dernier mouvement respiratoire observable de chaque souris. Ce dernier mouvement est considéré comme l'indice ultime d'une fonction du système nerveux central. L'arrêt respiratoire apparaît approximativement 19 secondes après l'injection de $MgCl_2$.

Des souris mâles (Charles River CD1) sont étudiées par groupes de 10.

Les souris sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule. Les rapports entre les modifications dans le terme de survie et la dose du composé sont enregistrés graphiquement selon une courbe semilogarithmique.

Cette courbe permet le calcul de la dose effective 3 secondes ($DE_{3''}$), c'est-à-dire le dose (en mg/kg) qui produit une augmentation de 3 secondes du temps de survie par rapport au groupe témoin de 10 souris non traitées.

Une augmentation de 3 secondes dans le temps de survie est à la fois significative statistiquement et reproductible.

Les $DE_{3''}$ des composés de l'invention vont de 6 à 60 mg/kg par voie i.p.

Les composés de l'invention ont également été soumis au test de l'hypoxie hypobare.

Des souris de souche CD1 sont maintenues dans une atmosphère appauvrie en oxygène, par réalisation d'un vide partiel (190 mm de mercure correspondant à 5,25 % d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100 %, est déterminée graphiquement. La DAM des composés de l'invention est de 10 à 32 mg/kg par voie intrapéritonéale.

Les composés de l'invention ont également fait l'objet d'un essai de fixation (binding) de $^3$H-imipramine sur le cortex total de rat.

La préparation du tissu comprend:

a) Une homogénéisation dans 50 volumes (par gramme de tissu) de tampon d'incubation, suivie d'une centrifugation à 20 000 t/mn pendant 10 mn.

b) Une répétition de l'opération précédente avec le culot de centrifugation.

c) Une reprise du culot final dans 33 volumes (par gramme de tissu) dans le tampon d'incubation, d'où une suspension de 30 mg de tissu par ml.

Le tampon d'incubation contient, par litre, 120 millimoles de chlorure de sodium, 50 millimoles de Tris et 5 millimoles de chlorure de potassium, et présente un pH de 7,4 à 0° C. Pour la détermination de la fixation totale, on introduit dans chaque tube d'incubation

100 µl de membrane de cortex à 30 mg/ml,
150 µl de tampon, et
50 µl de $^3$H-imipramine.

Pour la détermination de la fixation non spécifique, on introduit dans chaque tube d'incubation

100 µl de membrane de cortex à 30 mg/ml,
140 µl de tampon,
10 µl de désipramine à $30.10^{-4}$ moles/l, et
50 µl de $^3$H-imipramine.

Pour les déplacements par les substances à étudier, la concentration de $^3$H-imipramine est de 2,5 nM, et celles des substances actives vont jusqu'à 100 µM.

L'incubation est effectuée à 0° C pendant 1 heure.

Pour évaluer l'activité des composés on établit une courbe du pourcentage d'inhibition de la fixation spécifique $^3$H-imipramine en fonction de la concentration en drogue déplaçante. L'$IC_{50}$ est la concentration en drogue déplaçante qui inhibe 50 % de la fixation spécifique $^3$H-imipramine (détermination graphique) et à la

concentration de ligand tritié de 2,5 nM.

Les concentrations $IC_{50}$ des composés de l'invention se situent pour la plupart à moins de 100 μM, et descendent jusqu'à 0,01 μM pour certains d'entre eux.

Enfin les composés de l'invention ont fait l'objet d'un essai quant à leur inhibition de la capture de la noradrénaline et de la sérotonine dans une préparation de synaptosomes non purifiés d'hypothalamus de rats, essai inspiré de la méthode de Ross S.B. et Renyi A.L. (Acta Pharmacolog. Toxicol., 36, 382-394, 1975).

Chaque mélange d'incubation comprend 0,1 nmole de $^3$H-noradrénaline, 0,1 nmole de $^{14}$C-sérotonine, 100 μl de la préparation de synaptosomes non purifiés (correspondant à 10 mg de tissu cérébral initial), le composé à étudier, et 1,8 ml de tampon de Krebs-Henseleit (pH 7,4, 1,25 μmole de nialamide, 114 μmoles d'acide ascorbique, 59,5 μmoles d'EDTA disodique et 1111 μmoles de glucose pour 100 ml de tampon).

Le processus de capture est déclenché par l'addition des amines marquées, et l'incubation est effectuée pendant 5 mn dans des tubes de centrifugation maintenus à 37°C. On interrompt la réaction en filtrant le milieu d'incubation sur des filtres millipore MF (mélange d'esters de cellulose) de 0,45 μm (ref HAWP 02500) puis en rinçant avec 2,5 ml de sérum physiologique. On introduit ensuite les filtres secs qui ont retenu les synaptosomes dans des flacons à scintillation, on ajoute 10 ml de liquide scintillant (Aquasol-NEN). On laisse reposer pendant 2 heures au cours desquelles les filtres sont dissouts puis on mesure la radioactivité.

La capture effective est obtenue en retranchant la capture passive dans les mêmes conditions mais dans la glace.

L'inhibition de la capture est déterminée en pourcentage de la capture effective, avec et sans le composé à étudier, dans le même essai. La concentration $IC_{50}$, c'est-à-dire la concentration (en μM) qui provoque 50 % d'inhibition, est établie à partir d'une courbe logarithmique de la réponse en fonction de la concentration.

La concentration $IC_{50}$ des composés de l'invention va de 0,23 à 10 μM dans le cas de la noradrénaline et de 0,08 à 10 μM dans le cas de la sérotonine.

L'étude pharmacologique des composés de l'invention montre qu'ils possèdent une activité antianoxique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des encéphalopathies métaboliques, et pour le traitement des états dépressifs.

L'invention comprend, par conséquent, toutes compositions pharmaceutiques renfermant les composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 1 à 100 mg par voie parentérale et de 5 à 500 mg par voie orale.

**Revendications** pour les Etats Contractants: BE, CH, DE, FR, GB, IT, LI, LU, NL, SE.

1. Composés caractérisés en ce qu'ils répondent à la formule générale I

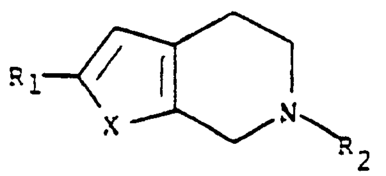

(I)

dans laquelle:

X représente un atome d'oxygène ou un groupe NH,

$R_1$ représente soit un groupe phényle portant éventuellement un ou deux atomes d'halogène ou un groupe méthyle, éthyle, trifluorométhyle, méthoxy, phényle ou, lorsque $R_2$ n'est pas un groupe benzyle, un groupe amino, soit un groupe naphtyle, soit un groupe benzodioxannyle, et

$R_2$ représente un atome d'hydrogène ou un groupe benzyle, ou bien encore, lorsque X représente un atome d'oxygène, $R_2$ peut représenter un groupe méthyle ou éthyle, ainsi que leurs sels d'addition à des acides acceptables en pharmacologie.

2. Composés selon la revendication 1, caractérisés en ce que, dans la formule I, $R_2$ représente un atome d'hydrogène.

3. Composés selon la revendication 1, caractérisés en ce que X représente un atome d'oxygène et $R_2$ représente un groupe méthyle ou éthyle.

4. Procédé de préparation des composés selon la revendication 1, caractérisé en ce qu'on cyclise un composé de formule II

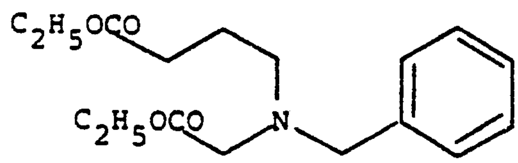

(II)

en présence d'un hydrure alcalin, puis on fait réagir le produit obtenu de formule III

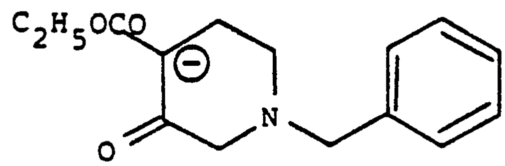

(III)

avec une acétophénone de formule IV

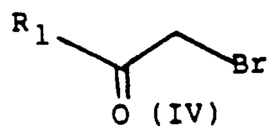

(IV)

dans laquelle $R_1$ est tel que défini à la revendication 1, pour obtenir un composé de formule V,

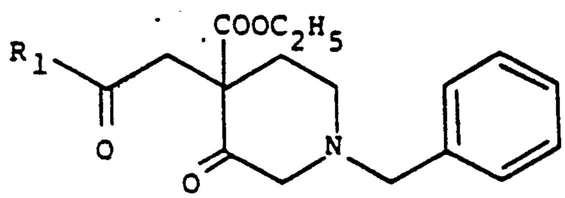

(V)

puis, selon le composé qu'on désire préparer,

- soit on effectue une cyclisation du composé (V) en présence d'acétate d'ammonium et d'acide acétique et on traite le composé obtenu, de formule VI,

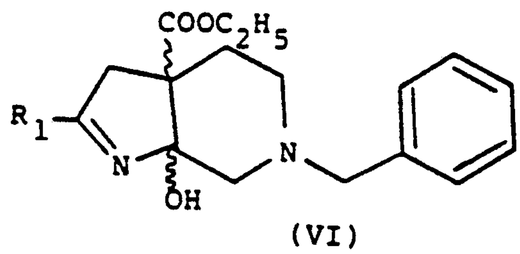

(VI)

d'abord avec de la soude puis avec de l'acide chlorhydrique,

- soit on effectue une cyclisation du composé (V) en présence d'un acide fort,

et finalement, si on le désire, on effectue une débenzylation du composé (I) ainsi obtenu pour obtenir un composé dans la formule duquel $R_2$ représente un atome d'hydrogène et, si on le désire, on effectue une alkylation pour obtenir un composé dans la formule duquel $R_2$ représente un groupe méthyle ou éthyle.

5. Composé selon la revendication 1 pour la préparation de médicaments.

6. Composition pharmaceutique caractérisée en ce qu'elle contient un composé selon la revendication 1, en association avec un excipient pharmaceutiquement acceptable.

**Revendication** pour pour l'Etat Contractant: AT.

Procédé de préparation des composés de formule générale I

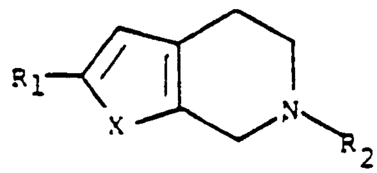

(I)

dans laquelle:

X représente un atome d'oxygène ou un groupe NH,

$R_1$ représente soit un groupe phényle portant éventuellement un ou deux atomes d'halogène ou un groupe méthyle, éthyle, trifluorométhyle, méthoxy, phényle ou, lorsque $R_2$ n'est pas un groupe benzyle, un groupe amino, soit un groupe naphtyle, soit un groupe benzodioxannyle, et

$R_2$ représente un atome d'hydrogène ou un groupe benzyle, ou bien encore, lorsque X représente un atome d'oxygène, $R_2$ peut représenter un groupe méthyle ou éthyle, procédé caractérisé en ce qu'on cyclise un composé de formule II

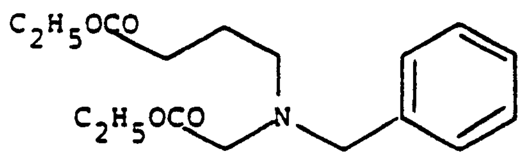

(II)

en présence d'un hydrure alcalin, puis on fait réagir le produit obtenu, de formule III

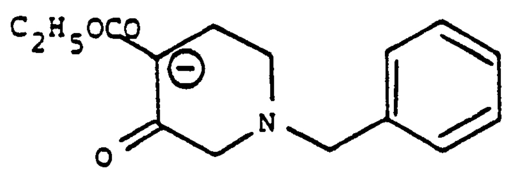

(III)

avec une acétophénone de formule IV

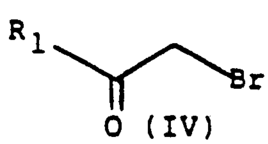

(IV)

pour obtenir un composé de formule V,

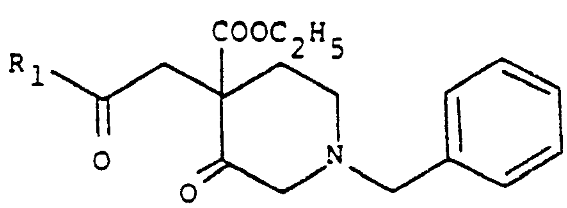

(V)

puis, selon le composé qu'on désire préparer,

- soit on effectue une cyclisation du composé (V) en présence d'acétate d'ammonium et d'acide acétique et on traite le composé obtenu, de formule VI,

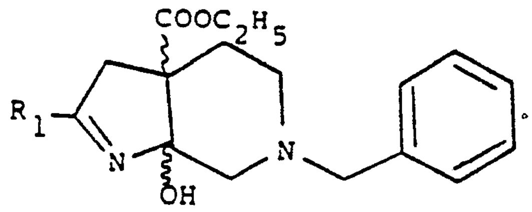

(VI)

d'abord avec de la soude puis avec de l'acide chlorhydrique,

- soit on effectue une cyclisation du composé (V) en présence d'un acide fort,

et finalement, si on le désire, on effectue une débenzylation du composé (I) ainsi obtenu pour obtenir un composé dans la formule duquel $R_2$ représente un atome d'hydrogène et, si on le désire, on effectue une alkylation pour obtenir un composé dans la formule duquel $R_2$ représente un groupe méthyle ou éthyle.

**Patentansprüche** für die Vertragsstaaten: BE, CH, DE, FR, GB, IT, LI, LU, NL, SE

1. Verbindungen, dadurch gekennzeichnet, daß sie der allgemeinen Formel I

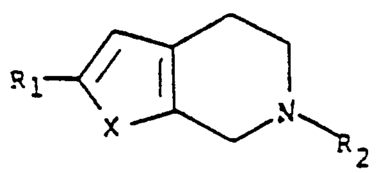

(I)

entsprechen, in welcher

X für eine Sauerstoffatom oder eine NH-Gruppe steht,

$R_1$ entweder einen gegebenenfalls durch ein oder zwei Halogenatome oder eine Methyl-, Ethyl-, Trifluormethyl-, Methoxy-, Phenyl- oder, wenn $R_2$ keine Benzylgruppe bedeutet, eine aminogruppe substituierte Phenylgruppe oder eine Naphthylgruppe oder eine Benzodioxanylgruppe darstellt und

$R_2$ für ein Wasserstoffatom oder eine Benzylgruppe steht oder aber, wenn X ein Bauerstoffatom bedeutet, $R_2$ eine Methyl- oder Ethylgruppe darstellen kann,

sowie deren Additionssalze mit pharmakologisch verwendbaren Säuren.

2. Verbindungen nach Anspruch 1, dadurch gekennzeichnet, daß $R_2$ in der Formel I ein Wasserstoffatom darstellt.

3. Verbindungen nach Anspruch 1, dadurch gekennzeichnet, daß X ein Bauerstoffatom und $R_2$ eine Methyl- oder Ethylgruppe darstellt.

4. Verfahren zur Herstellung von Verbindungen nach Anspruch 1, dadurch gekennzeichnet, daß man eine Verbindung der Formel II

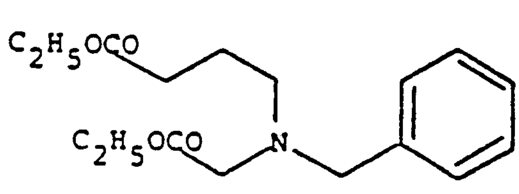

(II)

in Gegenwart eines Alkalihydrids zyklisiert, dann das erhaltene Produkt der Formel III

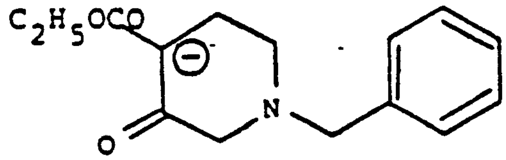

(III)

mit einem Acetophenon der Formel IV

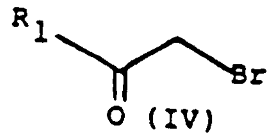

(IV)

in welcher $R_1$ die in Anspruch 1 angegebene Bedeutung hat, reagieren läßt, um eine Verbindung der Formel V

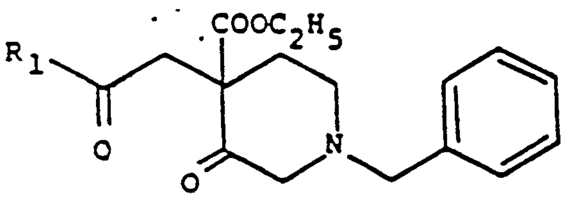

(V)

zu erhalten, worauf man, je nach der Verbindung, die man herzustellen wünscht,

- entweder eine Zyklisierung der Verbindung (V) in Gegenwart von Ammoniumacetat und Essigsäure hervorruft und die erhaltene Verbindung der Formel VI

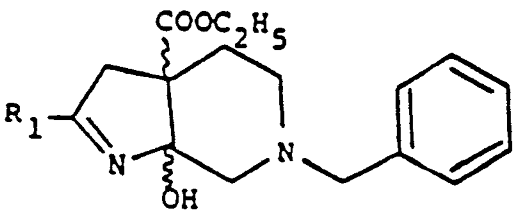

(VI)

zuerst mit Soda, dann mit Chlorwasserstoffsäure behandelt, oder

- eine Zyklisierung der Verbindung (V) in Gegenwart einer starken Säure hervorruft und

schließlich gewünschtenfalls eine Debenzylierung der so erhaltenen Verbindung (I) hervorruft, um eine Verbindung zu erhalten, in deren Formel $R_2$ ein Wasserstoffatom darstellt, und gewünschtenfalls eine Alkylierung durchführt, um eine Verbindung zu erhalten, in deren Formel $R_2$ eine Methyl- oder Ethylgruppe bedeutet.

5. Verbindung nach Anspruch 1, zur Herstellung von Arzneimitteln.

6. Pharmazeutische Zusammensetzung, dadurch gekennzeichnet, daß sie eine Verbindung nach Anspruch 1 in Kombination mit einem pharmazeutisch verwendbaren Träger enthält.

**Patentanspruch** für den Vertragsstaat: AT

Verfahren zur Herstellung von Verbindungen der allgemeinen Formel I

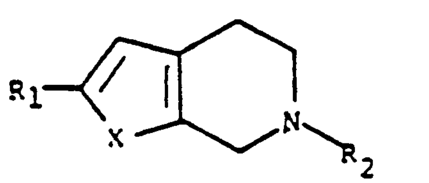

(I)

in welcher X für eine Sauerstoffatom oder eine NH-Gruppe steht,

$R_1$ entweder einen gegebenenfalls durch ein oder zwei Halogenatome oder eine Methyl-, Ethyl-, Trifluormethyl-, Methoxy-, Phenyl- oder, wenn $R_2$ keine Benzylgruppe bedeutet, eine Aminogruppe substituierte Phenylgruppe oder eine Naphthylgruppe oder eine Benzodioxanylgruppe darstellt und

$R_2$ für ein Wasserstoffatom oder eine Benzylgruppe steht oder aber, wenn X ein Sauerstoffatom bedeutet, $R_2$ eine Methyl- oder Ethylgruppe darstellen kann,

welches Verfahren dadurch gekennzeichnet ist, daß man ein, Verbindung der Formel II

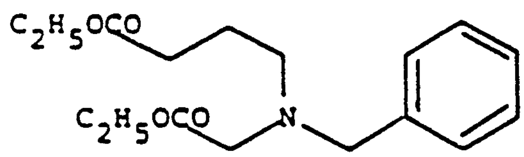

(II)

in Gegenwart eines Alkalihydrids zyklisiert dann das erhaltene Produkt der Formel III

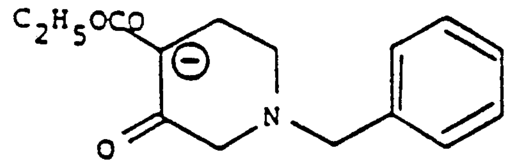

(III)

mit einem Scetophenon der Formel IV

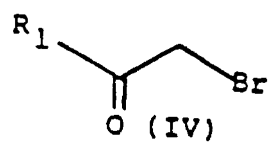

(IV)

reagieren läßt, um eine Verbindung der Formel V

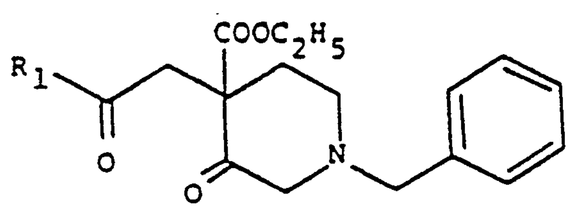

(V)

zu erhalten worauf man je nach der Verbindung, die man herzustellen wünscht,

- entweder eine Zyklisierung der Verbindung (V) in Gegenwart von Ammoniumacetat und Essigsäure hervorruft und die erhaltene Verbindung der Formel VI

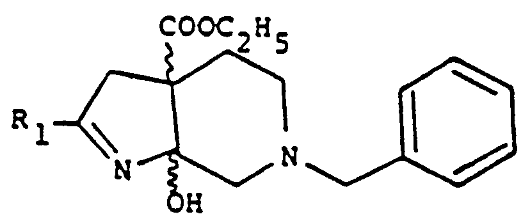

(VI)

zuerst mit Soda, dann mit Chlorwasserstoffsäure behandelt, oder

- eine Zyklisierung der Verbindung (V) in Gegenwart einer starken Säure hervorruft und schließlich gewünschtenfalls eine Debenzylierung der so erhaltenen Verbindung (I) hervorruft, um eine Verbindung zu erhalten, in deren Formel $R_2$ ein Wasserstoffatom darstellt, und gewünschtenfalls eine Alkylierung durchführt, um eine Verbindung zu erhalten, in deren Formel $R_2$ eine Methyl- oder Ethylgruppe bedeutet.

**Claims** for the contracting States: BE, CH, DE, FR, GB, IT, LI, LU, NL, SE.

1. Compounds characterised in that they correspond to the general formula I

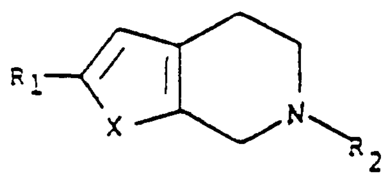

(I)

in which

X denotes an oxygen atom or an NH group,

$R_1$ either denotes a phenyl group optionally bearing one or two halogen atoms or a methyl, ethyl, trifluoromethyl, methoxy or phenyl group or, when $R_2$ is not a benzyl group, an amino group, or it denotes a naphthyl group or a benzodioxanyl group, and

$R_2$ denotes a hydrogen atom or a benzyl group, or alternatively, when X denotes an oxygen atom, $R_2$ can denote a methyl or ethyl group,

as well as their addition salts with acids which are acceptable in pharmacology.

2. Compounds according to Claim 1, characterised in that, in the formula I, $R_2$ denotes a hydrogen atom.

3. Compounds according to Claim 1, characterised in that X denotes an oxygen atom and $R_2$ denotes a methyl or ethyl group.

4. Process for preparing compounds according to Claim 1, characterised in that a compound of formula II

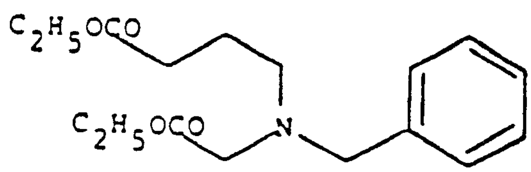

(II)

is cyclised in the presence of an alkali metal hydride, and the product obtained, of formula III

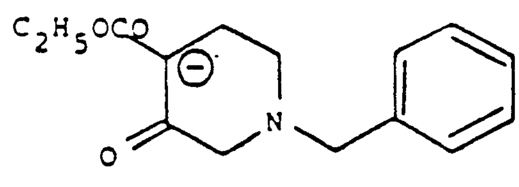

(III)

is reacted with an acetophenone of formula IV

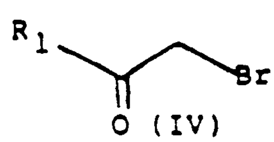

(IV)

in which $R_1$ is as defined in Claim 1, to obtain a compound of formula V

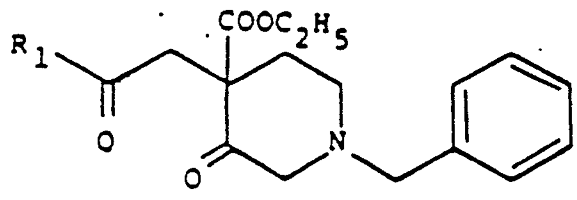

(V)

and then, depending on the compound which it is desired to prepare,

either the compound (V) is cyclised in the presence of ammonium acetate and acetic acid, and the compound obtained, of formula VI

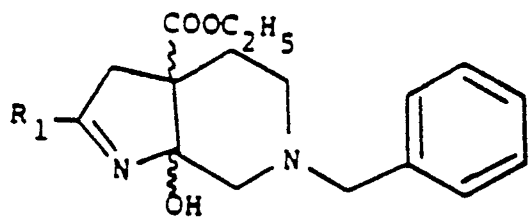

(VI)

is treated first with caustic soda and then with hydrochloric acid,

or the compound (V) is cyclise is an the precence of a strong acid,

and finally, if so desired, debenzylation of the compound (I) thus obtained is performed to obtain a compound in the formula of which $R_2$ denotes a hydrogen atom, and, if so desired, alkylation is performed to obtain a compound in the formula of which $R_2$ denotes a methyl or ethyl group.

5. Compound according to Claim 1 for the preparation of drugs.

6. Pharmaceutical composition, characterised in that it contains a compound according to Claim 1, in combination with a pharmaceutically acceptable excipient.

**Claim** for the contracting State: AT

Process for preparing compounds of general formula I

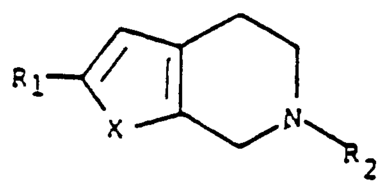

(I)

in which

X denotes an oxygen atom or an NH group,

$R_1$ either denotes a phenyl group optionally bearing one or two halogen atoms or a methyl, ethyl, trifluoromethyl, methoxy or phenyl group or, when $R_2$ is not a benzyl group, an amino group, or it denotes a naphthyl group or a benzodioxanyl group, and

$R_2$ denotes a hydrogen atom or a benzyl group, or alternatively, when X denotes an oxygen atom, $R_2$ can denote a methyl or ethyl group,

which process is characterised in that a compound of formula

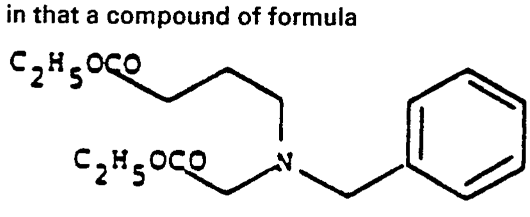

(II)

is cyclised in the presence of an alkali metal hydride, and the product obtained, of formula

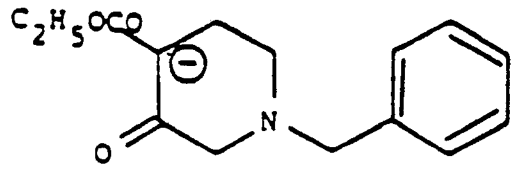

(III)

is reacted with an acetophenone of formula IV

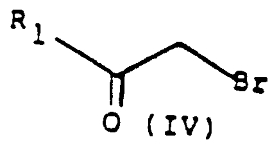

(IV)

to obtain a compound of formula V

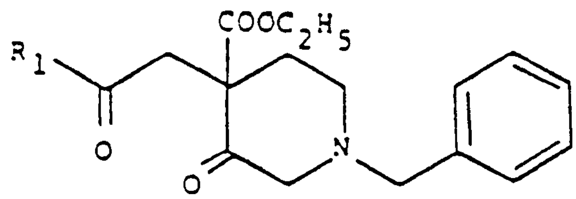

(V)

and then, depending on the compound which it is desired to prepare,

either the compound (V) is cyclised in the presence of ammonium acetate and acetic acid, and the compound obtained, of formula VI

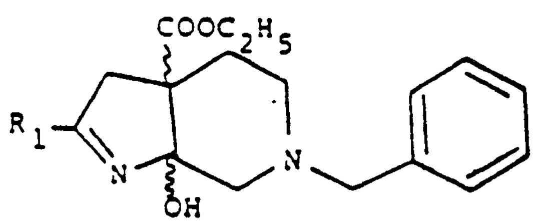

(VI)

is treated first with caustic soda and then with hydrochloric acid,

or the compound (V) is cyclised in the presence of a strong acid,

and finally, if so desired, debenzylation of the compound (I) thus obtained is performed to obtain a compound in the formula of which $R_2$ denotes a hydrogen atom, and, if so desired, alkylation is performed to obtain a compound in the formula of which $R_2$ denotes a methyl or ethyl group.